# EUROPEAN PATENT APPLICATION

(11) **EP 2 338 406 A1**
(43) Date of publication of application: **29.06.2011**
(21) Application number: 10009617.1
(22) Date of filing: 15.09.2010
(51) Int. Cl.: A61B 1/267, A61B 1/04

(54) **Stylet with a camera device**

(30) Priority: 24.12.2009 TW 98224241 U
(71) Applicant: Chen, Tien-Sheng, 112 Taipei City (TW)
(72) Inventor: Chen, Tien-Sheng, 112 Taipei City (TW)
(74) Representative: Altenburg, Bernardus Stephanus Franciscus

(57) **Abstract**

A stylet with a camera device is disclosed, which is used along with an endotracheal tube. The stylet with a camera device comprises: a tube body, placed in the endotracheal tube, the tube body having a front end and a rear end, the rear end of the tube body having a bending part; an image capturing unit, used for capturing an image, the image capturing unit being connected to the front end of the tube body; a display unit, used for displaying the image, the display unit being connected to the bending part, and the display unit being electrically connected to the image capturing unit, wherein the display unit is located in a rear side of the endotracheal tube; and an operating member, connected to the rear end or the display unit, wherein the operating member allows a user to apply force so as to make the front end of the tube body protrude from one end of the endotracheal tube.

## Description

### BACKGROUND OF THE INVENTION

### 1. Field of the Invention

The present invention relates to a stylet with a camera device; more particularly, the present invention relates to a stylet with a camera device capable of being used along with an endotracheal tube.

### 2. Description of the Related Art

During a general anesthesia procedure, an endotracheal intubation procedure must be performed to keep an anesthetized patient breathing normally. In most cases, in order to prevent the anesthetized patient from suffering from an oxygen deficit, the medical personnel must insert an endotracheal tube into the anesthetized patient's trachea within a very short amount of time during the intubation procedure. Therefore, it is the primary task of the medical personnel to rapidly and effectively perform the intubation procedure during the general anesthesia procedure.

However, because anesthetized patients of different ages and figures may have different upper airway structures, sometimes it is not easy for the medical personnel to directly observe the position of the anesthetized patient's trachea. Therefore, in practice, the medical personnel often utilize an auxiliary tool to help facilitate the intubation procedure. For example, the medical personnel may first press a laryngoscope against the anesthetized patient's tongue base, and then insert a stylet with a camera device into the anesthetized patient's upper airway so as to observe the position of the trachea. Because the endotracheal tube has been sleeved onto the stylet in advance, once the stylet determines the position of the trachea, the endotracheal tube can be rapidly pushed into the trachea. The above practice not only eliminates time wasted by medical personnel blindly searching for the position of the trachea but also prevents damage to the anesthetized patient's upper airway tissue during the intubation procedure.

However, although the above conventional method of utilizing the stylet along with the endotracheal tube can facilitate the intubation procedure, it still has some disadvantages. For example, when the medical professional is using the device, he/she has to concentrate on the front end of the stylet while constantly adjusting the angle of a display at the same time in order to directly observe the result from the display, which increases both difficulty of operation and consumption of time, further raising the amount of risk to the patient.

In known prior arts, although a stylet with a camera device which can be operated by a single hand has been provided (such as Trachway™, which awarded iF product design award in 2007, http://www.airwaydevices.com.tw/upload/product/Trachway-%E8%8B%B1%E6%96%87_PDF.pdf), the tube body of the stylet is long-shaped, and its display monitor is connected to a remote position of the tube body. Because the display monitor is farther away from a user, it is not easy for the user to observe the result clearly. The medical professional not only has to extend his/her arm straight out, but also needs to move his/her wrist, elbow or even shoulder joints during operation, which is less flexible and accurate, thereby resulting in operation inconvenience.

Further, during the intubation procedure, as the front end of the stylet moves along the arc-shaped curve of the oral cavity, the angle of the stylet changes, and the tube body gradually changes from a horizontal direction to a vertical direction, such that the display monitor changes its position accordingly. As a result, front of the display monitor does not continuously face toward the user, and the user has to constantly adjust the display with a finger (such as by adjusting a control button) to keep the display monitor in a direct-view status.

Therefore, there is a need to provide a stylet with a camera device to increase precision during operation, so as to mitigate and/or obviate the aforementioned problems.

### SUMMARY OF THE INVENTION

It is an object of the present invention to provide a stylet with a camera device, which is used along with an endotracheal tube. The stylet with a camera device is capable of keeping its display unit facing in the same direction during operation, and the display unit is closer to a user.

To achieve the abovementioned object, the stylet with a camera device of the present invention comprises: a tube body, placed in the endotracheal tube, the tube body having a front end and a rear end, the rear end of the tube body having a bending part; an image capturing unit, used for capturing an image, the image capturing unit being connected to the front end of the tube body; a display unit, used for displaying the image, the display unit being connected to the bending part, and the display unit being electrically connected to the image capturing unit, wherein the display unit is located in a rear side of the endotracheal tube; and an operating member, connected to the rear end or the display unit, wherein the operating member allows a user to apply force so as to make the front end of the tube body protrude from a first end of the endotracheal tube.

Other objects, advantages, and novel features of the invention will become more apparent from the following detailed description when taken in conjunction with the accompanying drawings.

### BRIEF DESCRIPTION OF THE DRAWINGS

These and other objects and advantages of the present invention will become apparent from the following description of the accompanying drawings, which disclose several embodiments of the present invention. It is to be understood that the drawings are to be used for purposes of illustration only, and not as a definition of the invention.

In the drawings, wherein similar reference numerals denote similar elements throughout the several views:
FIG. 1 illustrates a schematic drawing showing a front end of a tube body of a stylet with a camera device just inserted into a patient's mouth according to one embodiment of the present invention.
FIG. 2 illustrates a schematic drawing showing the front end of the tube body of the stylet with a camera device inserted close to the patient's trachea according to one embodiment of the present invention.
FIG. 3 illustrates a schematic drawing showing the front end of the tube body of the stylet with a camera device protruded from an endotracheal tube according to one embodiment of the present invention.
FIG. 3A illustrates a schematic drawing of an operating member of the stylet with a camera device according to another embodiment of the present invention.

### DETAILED DESCRIPTION OF THE PREFERRED EMBODIMENT

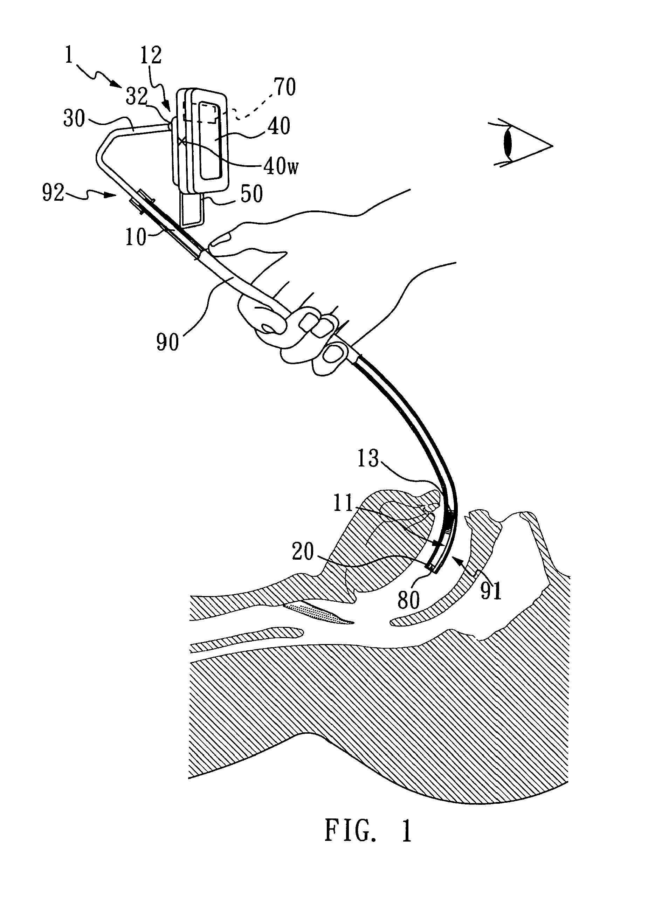

Please refer to FIGs. 1 to 3, which illustrate a stylet with a camera device according to one embodiment of the present invention.

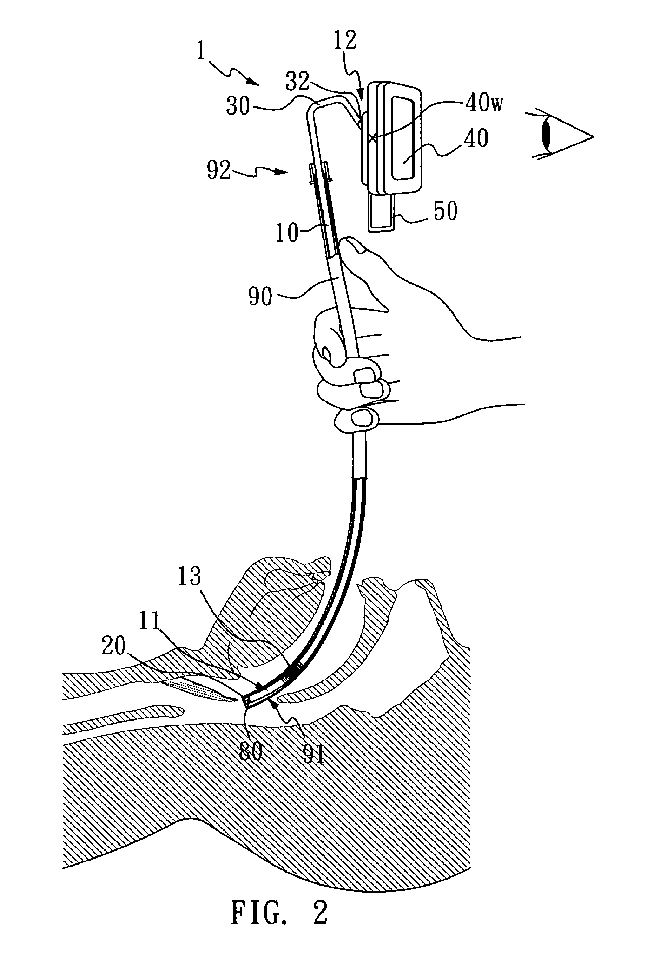

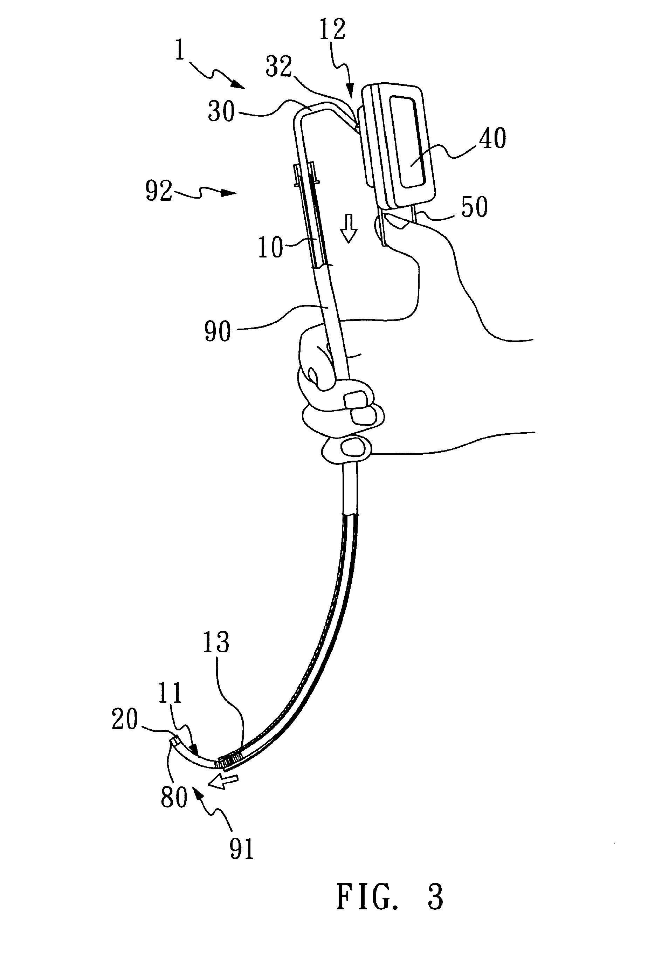

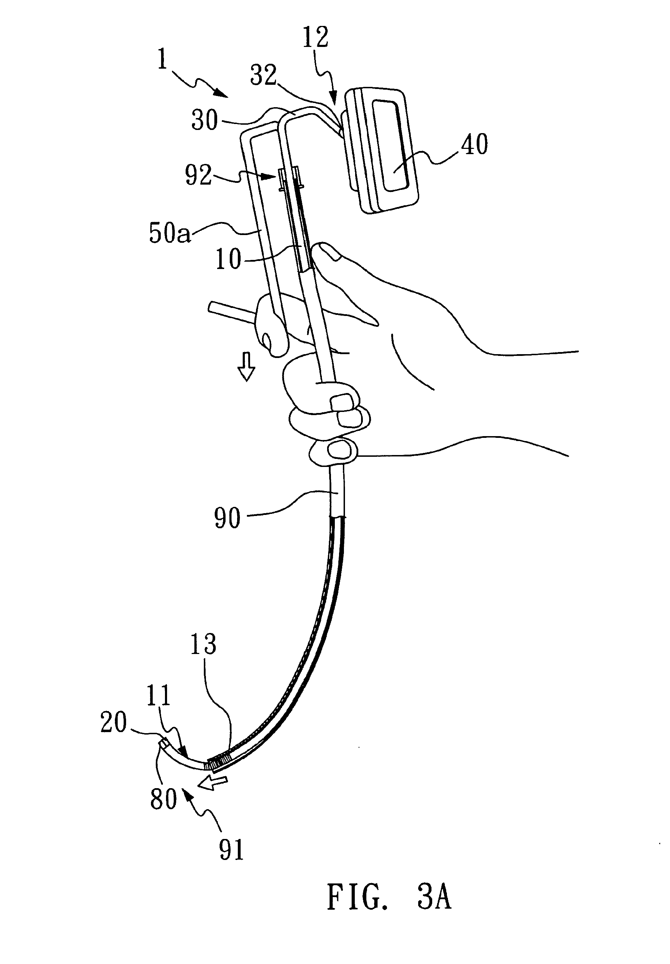

FIG. 1 illustrates a schematic drawing showing a front end of a tube body just inserted into a patient's mouth; FIG. 2 illustrates a schematic drawing showing the front end of the tube body inserted close to the patient's trachea; and FIG. 3 illustrates a schematic drawing showing the front end of the tube body protruding from an endotracheal tube.

The stylet with a camera device 1 of the present invention is used along with an endotracheal tube 90. The endotracheal tube 90 comprises a first end 91 and a second end 92. The stylet with a camera device 1 comprises a tube body 10, an image capturing unit 20, a display unit 40, an operating member 50, a power supply unit 70, and a light-emitting unit 80.

When the stylet with a camera device 1 is under operation, most of the tube body 10 is placed in the endotracheal tube 90. The tube body 10 has a front end 11 and a rear end 12. The front end 11 of the tube body 10 has an elastic section 13, and the rear end 12 of the tube body 10 has a bending part 30. The rear end 12 of the tube body 10 is not always extended upwards; the rear end 12 of the tube body 10 can be bent via the bending part 30 so as to face in different directions.

The image capturing unit 20 is used for capturing an image. The image capturing unit 20 is connected to the front end 11 of the tube body 10. The light-emitting unit 80 is also installed in the front end 11 of the tube body 10, and is used for illuminating an area that the image capturing unit 20 is going to shoot.

The display unit 40 is used for displaying the image captured by the image capturing unit 20. The display unit 40 is connected to the bending part 30, and the display unit 40 is electrically connected to the image capturing unit 20. Please note that the display unit 40 is not located in the top position extended from the endotracheal tube 90 as described in the known prior arts. In this invention, the display unit 40 is located in a rear side (i.e. the side closer to a doctor or other medical professional) of the endotracheal tube 90 via the bending part 30. Due to gravity, during the whole procedure of intubation, the screen of the display unit 40 substantially faces toward the user.

The power supply unit 70 is respectively electrically connected to the image capturing unit 20, the display unit 40, and the light-emitting unit 80. The power supply unit 70 supplies power to the image capturing unit 20, the display unit 40, and the light-emitting unit 80 for normal operation.

In this embodiment, the bending part 30 and the display unit 40 are flexibly jointed to each other. The bending part 30 is pivoted to the display unit 40 at a pivot point 32, and the pivot point 32 of the bending part 30 and the display unit 40 is higher than the center-of-gravity position 40w of the display unit 40. Therefore, according to the weight of the display unit 40 itself, when an operating angle of the tube body 10 changes, because of the gravity, the direction of the display unit 40 is adjusted automatically without manual control such that the display unit 40 still faces toward the same direction (in this embodiment, it faces toward a horizontal direction), improving the drawback of being adjusted by a user's finger in the prior art (such as Trachway™) .

Comparing FIG. 1 and FIG. 2, although the tube bodies 10 are at different operating angles, the gravity of the center-of-gravity position 40w of the display unit 40 always faces downwards,.such that a moment is generated between the gravity and the pivot point 32, so as to make the display unit 40 slightly rotate (for example, when the status changes from FIG. 1 to FIG. 2, the display unit 40 will rotate clockwise), thereby making a displayed screen of the display unit 40 always face the same direction (for example, the display units 40 shown in FIG. 1 and FIG. 2 both face in the horizontal direction). Therefore, the display unit 40 can face toward the user without the need for the user to pay additional attention to operating other adjustment members, such that the user can view the image captured by the image capturing unit 20 at any time.

The operating member 50 allows the user to apply force downwards, so as to make the front end 11 of the tube body 10 protrude from the first end 91 of the endotracheal tube 90 (as shown in FIG. 3). Meanwhile, at least one part of the elastic section 13 located in the front end 11 is protruded from the endotracheal tube 90. According to the function of the elastic section 13, the tube body 10 protruded from the endotracheal tube 90 will have a bigger curved radian, so as to facilitate the procedure of successfully placing the front end 11 of the tube body 10 into the patient's trachea by means of bypassing the epiglottic cartilage.

In this embodiment, the operating member 50 is connected to a lower rim of the display unit 40, and the operating member 50 is a ring-shaped body which allows the user to use his/her thumb to apply force downwards (as the arrow direction shown in FIG. 3).

Please note that the position and shape of the operating member 50 are not limited to the above description. Please refer to FIG. 3A, which illustrates a schematic drawing of the operating member of the stylet with a camera device according to another embodiment of the present invention. In this embodiment, the operating member 50a is connected to the bending part 30, and the operating member 50a is a hook-shaped body which allows the user to use his/her index finger to apply force downwards. The operating member can also be an adjustment knob.

According to the above description, in the stylet with a camera device of the present invention, by means of benefiting from the bending part of the tube body, the display unit connected to the bending part can be positioned closer to the user. Furthermore, the stylet with a camera device allows the user to rotate only his/her wrist during the operation of the display unit so as to make the operation more accurate. Moreover, the display unit can automatically change the angle between the display unit and the tube body due to gravity; therefore, the display unit can always face toward the user without the need for the user to pay additional attention to controlling it, such that the medical personnel can constantly monitor the procedure of the endotracheal tube and stylet passed through the patient's mouth and larynx and finally inserted into the trachea.

Although the present invention has been explained in relation to its preferred embodiments, it is to be understood that many other possible modifications and variations can be made without departing from the spirit and scope of the invention as hereinafter claimed.

## Claims

1. A stylet with a camera device, suitable for use with an endotracheal tube wherein the endotracheal tube comprises a first end and a second end, the stylet with a camera device comprising:
a tube body, capable of being inserted in the endotracheal tube, the tube body having a front end and a rear end, the rear end of the tube body having a bending part;
an image capturing unit, capable of capturing an image, the image capturing unit being connected to the front end of the tube body and capable of being passed through the endotracheal tube from the second end towards and beyond the first end of the endotracheal tube;
a display unit, used for displaying the image, the display unit being connected to the rear end, the display unit being electrically connected to the image capturing unit, wherein when the stylet is fully inserted in the endotracheal tube the display unit is located outside the endotracheal tube at the second end of the endotracheal tube; and
an operating member, connected to the rear end or the display unit, wherein the operating member allows a user to apply force so as to make the front end of the tube body protrude from the first end of the endotracheal tube.

2. The stylet with a camera device as claimed in claim 1, wherein the bending part is pivoted to the display unit at a pivot point, and the pivot point of the bending part and the display unit is higher than the center-of-gravity position of the display unit.

3. The stylet with a camera device as claimed in claim 1 or 2, wherein the operating member is a ring-shaped body or a hook-shaped body.

4. The stylet with a camera device as claimed in any of claims 1 to 3, wherein the bending part and the display unit are flexibly jointed to each other.

5. The stylet with a camera device as claimed in any of claims 1 to 4, wherein the direction of the display unit can be adjusted automatically by the gravity.

6. The stylet with a camera device as claimed in any of the preceding claims, wherein the front end of the tube body has an elastic section.

7. The stylet with a camera device as claimed in any of the preceding claims further comprising:
a power supply unit, respectively electrically connected to the image capturing unit and the display unit; and
a light-emitting unit, installed in the front end, the light-emitting unit being electrically connected to the power supply unit.

8. The stylet with a camera device as claimed in any of the preceding claims, wherein the operating member is connected to the bending part.
